## Europäisches Patentamt
## European Patent Office
## Office européen des brevets

(19)

(11) Veröffentlichungsnummer : **0 016 474**
**B1**

(12) EUROPÄISCHE PATENTSCHRIFT

(45) Veröffentlichungstag der Patentschrift :
24.02.82

(51) Int. Cl.³ : **C 07 C 7/08**, C 07 C 11/12

(21) Anmeldenummer : 80101530.6

(22) Anmeldetag : 22.03.80

(54) **Verfahren zur Gewinnung eines konjugierten Diolefins aus einem C$_4$- oder C$_5$-Kohlenwasserstoffgemisch.**

(30) Priorität : 23.03.79 DE 2911394

(43) Veröffentlichungstag der Anmeldung :
01.10.80 (Patentblatt 80/20)

(45) Bekanntmachung des Hinweises auf die Patenterteilung : 24.02.82 Patentblatt 82/08

(84) Benannte Vertragsstaaten :
AT BE DE FR GB IT NL SE

(56) Entgegenhaltungen :
DE - A1 - 2 401 102
DE - B - 2 006 863

(73) Patentinhaber : BASF Aktiengesellschaft
Carl-Bosch-Strasse 38
D-6700 Ludwigshafen (DE)

(72) Erfinder : Volkamer, Klaus, Dr.
Heidelberger Ring 21
D-6710 Frankenthal (DE)
Erfinder : Broellos, Klaus, Dr.
Florlansring 7
D-6101 Seeheim (DE)
Erfinder : Lindner, Alfred, Dr.
Ringstrasse 22
D-6712 Bobenheim-Roxheim 1 (DE)
Erfinder : Wagner, Ulrich, Dr.
Knospstrasse 7
D-6703 Limburgerhof (DE)
Erfinder : Weitz, Hans-Martin, Dr.
Auf dem Koeppel 40
D-6702 Bad Duerkheim 1 (DE)
Erfinder : Schneider, Klaus, Juergen, Dr.
Triftbrunnenweg 16
D-6730 Neustadt 19 (DE)

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

## Verfahren zur Gewinnung eines konjugierten Diolefins aus einem $C_4$- oder $C_5$-Kohlenwasserstoffgemisch

Die vorliegende Erfindung betrifft ein Verfahren zur Gewinnung eines konjugierten Diolefins aus einem $C_4$- oder $C_5$-Kohlenwasserstoffgemisch durch extraktive Destillation mit Hilfe eines selektiven Lösungsmittels.

Die extraktive Destillation ist ein bekanntes Verfahren zur Trennung von Gemischen, die durch übliche fraktionierte Destillation nicht leicht trennbar sind, z.B. wenn die zu trennenden Komponenten ein Azeotrop bilden oder geringe Unterschiede in den relativen Flüchtigkeiten besitzen. Bei der extraktiven Destillation wird in die Destillationskolonne eine solche Menge eines relativ schwer flüchtigen Lösungsmittels eingeführt, daß die Unterschiede in den relativen Flüchtigkeiten der zu trennenden Komponenten erhöht werden und damit eine destillative Trennung ermöglicht wird. Typische Anwendungsbeispiele für die extraktive Destillation finden sich beispielsweise in C.S. Robinson et al « Elements of Fractional Distillation » 4. Auflage McGraw-Hill Book Company, Inc., New York (1959), Seite 291.

Es ist bekannt, z.B. aus der DE-AS 15 68 902, DE-PS 11 63 795 oder aus The Soviet Chemical Industry, Nr. 11, November 1971, Seiten 719 bis 723, konjugierte Diolefine aus einem $C_4$- oder $C_5$-Kohlenwasserstoffgemisch durch extraktive Destillation unter Verwendung eines selektiven Lösungsmittels zu gewinnen. Die selektiven Lösungsmittel können weitgehend wasserfrei verwendet werden. Bei dieser Arbeitsweise, die besonders bei hydrolyse-empfindlichen Lösungsmitteln angewendet wird, ist jedoch im allgemeinen die $C_4$- oder $C_5$-Kohlenwasserstoff-Selektivität nicht ausreichend. Den selektiven Lösungsmitteln ist daher zur Erhöhung der Selektivität und zur Erniedrigung des Siedepunkts Wasser zugemischt worden. Die Wasserzugabe zum selektiven Lösungsmittel hat jedoch den Nachteil, daß die Löslichkeit der $C_4$- oder $C_5$-Kohlenwasserstoffe im selektiven Lösungsmittel erniedrigt wird, so daß die Menge an umlaufendem selektivem Lösungsmittel in der Extraktionsanlage entsprechend erhöht ist.

Die vorliegende Erfindung soll nun eine Verbesserung der Arbeitsweise und Wirtschaftlichkeit der bekannten Verfahren bewirken.

Es wurde nun ein vorteilhaftes Verfahren gefunden zur Gewinnung eines konjugierten Diolefins aus einem dieses enthaltenden $C_4$- oder $C_5$-Kohlenwasserstoffgemisch durch ein- oder mehrstufige extraktive Destillation mit einem selektiven Lösungsmittel, welches dadurch gekennzeichnet ist, daß man als selektives Lösungsmittel eine Lösungsmittelmischung verwendet, die

a) 1 bis 99 Gewichtsprozent eines N-alkylsubstituierten niederen aliphatischen Säureamids oder eines N-alkylsubstituierten alicyclischen Säureamids mit 5 Ringgliedern und

b) 1 bis 99 Gewichtsprozent eines niederen aliphatischen Carbonsäureesters oder Kohlensäureesters mit einem Siedepunkt im Bereich von 30 °C bis 200 °C enthält.

Die Löslichkeit der $C_4$- oder $C_5$-Kohlenwasserstoffe in der erfindungsgemäß als selektives Lösungsmittel zu verwenden Lösungsmittelmischung ist gegenüber den bekannten Verfahren bei vergleichbar guter $C_4$- oder $C_5$-Kohlenwasserstoff-Selektivität wesentlich erhöht, so daß die Menge an umlaufendem selektivem Lösungsmittel in der Extraktionsanlage zur Gewinnung des konjugierten Diolefins stark erniedrigt werden kann. Dies hat insbesondere eine starke Senkung der Investitionskosten für die Extraktionsanlage und der Verbräuche an Dampf und elektrischer Energie zur Folge. Außerdem weist die erfindungsgemäß zu verwendende Lösungsmittelmischung eine geringere Viskosität, eine geringere Verdampfungswärme und eine geringere spezifische Wärme als die bekannten selektiven Lösungsmittel mit vergleichbar guter $C_4$- oder $C_5$-Kohlenwasserstoff-Selektivität auf. Die geringere Viskosität der erfindungsgemäß zu verwendenden Lösungsmittelmischung bewirkt einen besseren Bodenwirkungsgrad in den extraktiven Destillationskolonnen, während durch die geringere Verdampfungswärme und geringere spezifische Wärme zusätzlich Energie eingespart werden kann.

Bei Verwendung einer Mischung eines der relativ hoch siedenden Lösungsmittel gemäß der vorstehenden Ziffer a) mit einem niedriger als dieses siedenden Lösungsmittel gemäß vorstehender Ziffer b), z.B. einem niedriger als 150 °C, vorzugsweise niedriger als 120 °C, siedenden Lösungsmittel gemäß vorstehender Ziffer b) wird zusätzlich als Vorteil erhalten, daß die beispielsweise als Ausgaser oder als Lösungsmittelabstreifer betriebene Lösungsmittelwiedergewinnungszone der Extraktionsanlage zur Gewinnung des konjugierten Diolefins bei jeweils gleicher Sumpftemperatur bei höherem Druck als mit den reinen unter Ziffer a) genannten Lösungsmitteln nach den bekannten Verfahren betrieben werden kann. Dies hat beispielsweise den Vorteil, daß die in der Lösungsmittelgewinnungszone erhaltenen ausgegasten Kohlenwasserstoffe aufgrund des höheren Drucks in einfacher Weise ohne Zwischenschaltung eines Kompressors oder Gebläses in nachgeschaltete, unter erhöhtem Druck betriebene Zonen gefördert werden können. Ein anderer Vorteil der Verwendung einer Mischung eines der relativ hoch siedenden Lösungsmittel gemäß der vorstehenden Ziffer a) mit einem niedriger als dieses siedenden Lösungsmittel gemäß vorstehender Ziffer b) besteht darin, daß die z.B. als Ausgaser oder als Lösungsmittelabstreifer betriebene Lösungsmittelwiedergewinnungszone der Extraktionsanlage bei Anwendung des gleichen Drucks wie nach den bekannten Verfahren bei einer niedrigeren Sumpftemperatur betrieben werden kann, so daß eine Verschmutzung der Extraktionsanlage durch Polymerbildung leichter vermieden werden kann.

Für das Verfahren nach der Erfindung wird eine Lösungsmittelmischung verwendet, die

a) 1 bis 99 Gewichtsprozent eines N-alkylsubstituierten niederen aliphatischen Säureamids oder eines N-alkylsubstituierten alicyclischen Säureamids mit 5 Ringgliedern und

b) 1 bis 99 Gewichtsprozent eines niederen aliphatischen Carbonsäureesters oder Kohlensäureesters mit einem Siedepunkt im Bereich von 30 °C bis 200 °C, vorzugsweise 40 °C bis 150 °C, insbesondere 50 °C bis 120 °C enthält.

Geeignete N-alkylsubstituierte niedere aliphatische Säureamide sind z.B. Dimethylformamid, Diäthylformamid, Dimethylacetamid, Diäthylacetamid, Formylmorpholin. Als N-alkylsubstituierte alicyclische Säureamide (Lactame) kommen z.B. N-Alkylpyrrolidone, insbesondere N-Methylpyrrolidon, in Betracht. Im allgemeinen werden N-alkylsubstituierte niedere aliphatische Säureamide oder N-alkylsubstituierte alicyclische Säureamide mit Siedepunkten im Bereich von 150 °C bis 260 °C, vorzugsweise im Bereich von 150 °C bis 210 °C, verwendet. Mit besonderem Vorteil werden von den Lösungsmitteln gemäß vorstehender Ziffer a) Dimethylformamid und insbesondere N-Methylpyrrolidon verwendet.

Die erfindungsgemäß zu verwendende Lösungsmittelmischung enthält 1 bis 99 Gewichtsprozent, vorzugsweise 2 bis 50 Gewichtsprozent, insbesondere 3 bis 20 Gewichtsprozent, an einem der Lösungsmittel gemäß vorstehender Ziffer b). Entsprechend weist die Lösungsmittelmischung einen Gehalt von 1 bis 99 Gewichtsprozent, vorzugsweise 50 bis 98 Gewichtsprozent, insbesondere 80 bis 97 Gewichtsprozent, an einem der Lösungsmittel gemäß vorstehender Ziffer a) auf. Bei der Verwendung einer N-Methylpyrrolidon enthaltenden Lösungsmittelmischung weist diese Mischung mit besonderem Vorteil einen Gehalt an einem der Lösungsmittel gemäß vorstehender Ziffer b) von 7 bis 17 Gewichtsprozent auf.

Als niedere aliphatische Carbonsäureester kommen beispielsweise solche in Betracht, die sich ableiten von niederen Monocarbonsäuren mit im allgemeinen 1 bis 4, vorzugsweise 1 bis 3 Kohlenstoffatomen, wie Ameisensäure, Essigsäure, Propionsäure oder von Dicarbonsäuren mit 2 oder 3 Kohlenstoffatomen, wie Oxalsäure oder Malonsäure. Die Alkohol-Komponente der Carbonsäureester leitet sich im allgemeinen ab von einwertigen Alkoholen mit zweckmäßig 1 bis 6, vorzugsweise 1 bis 4 Kohlenstoffatomen, wie Methanol, Äthanol, Propanol, Isopropanol, n-Butanol, Isobutanol oder von zweiwertigen Alkoholen mit im allgemeinen 2 oder 3 Kohlenstoffatomen, wie Äthylenglykol, 1,2-Propylenglykol. Bei den sich von zweiwertigen Alkoholen ableitenden Carbonsäureestern kommen der Monoester und vorzugsweise der Diester in Betracht. Geeignete niedere aliphatische Carbonsäureester sind z.B. Ameisensäureester, wie Ameisensäuremethylester, -äthylester, -n-propylester, -isopropylester, -n-butylester, -isobutylester, -amylester, -hexyl-

ester, Äthylenglykoldiformiat, Äthylenglykolmonoformiat. Essigsäureester, wie Essigsäuremethylester, -äthylester, -n-propylester, -isopropylester, -n-butylester, -isobutylester, -amylester, -hexylester, Äthylenglykoldiacetat; Propionsäureester, wie Propionsäuremethylester, -äthylester, -n-propylester, -isopropylester, -n-butylester, -isobutylester, -amylester, -hexylester; Oxalsäureester, wie Oxalsäuredimethylester, -diäthylester, Malonsäureester, wie Malonsäuredimethylester, -diäthylester. Als Kohlensäureester kommen die unsymmetrischen und vorzugsweise die symmetrischen Ester in Betracht, die sich im allgemeinen von einwertigen Alkoholen mit 1 bis 4 Kohlenstoffatomen ableiten, wie Methanol, Äthanol, Propanol, Isopropanol, n-Butanol, Isobutanol. Geeignete Kohlensäureester sind z.B. der Kohlensäure-dimethylester, -diäthylester, -diisopropylester, -di-n-propylester, -diisobutylester, -di-n-butylester. Neben den reinen Estern können auch ihre Mischungen, bei Diestern von zweiwertigen Alkoholen auch ihre Mischungen, die noch Monoester enthalten, verwendet werden.

Die erfindungsgemäß zu verwendende Lösungsmittelmischung kann einen geringen Wassergehalt, z.B. bis zu 10 Gewichtsprozent, aufweisen. Zweckmäßig wird jedoch der Wassergehalt auf höchstens 5 Gewichtsprozent, vorzugsweise höchstens 3 Gewichtsprozent bezogen auf die Lösungsmittelmischung begrenzt. Es kann jedoch auch vorteilhaft sein, im wesentlichen wasserfrei, d.h. mit einem Wassergehalt von höchstens 1 Gewichtsprozent, vorzugsweise höchstens 0,5 Gewichtsprozent, insbesondere höchstens 0,1 Gewichtsprozent, bezogen auf die Lösungsmittelmischung, zu arbeiten.

Die Gewinnung eines konjugierten Diolefins aus einem dieses enthaltenden $C_4$- oder $C_5$-Kohlenwasserstoffgemisch unter Verwendung der erfindungsgemäß als selektives Lösungsmittel einzusetzenden Lösungsmittelmischung erfolgt in an sich bekannter Weise (vgl. z.B. DE-PS 11 84 334, DE-AS 15 68 876, DE-AS 15 68 902) durch ein- oder mehrstufige, zweckmäßig ein oder zweistufige extraktive Destillation. Die Gewinnung der konjugierten Diolefine, wie 1,3-Butadien, Isopren und 1,3-Pentadien aus der $C_4$- oder $C_5$-Kohlenwasserstoffmischung erfolgt beispielsweise in der Weise, daß das $C_4$- oder $C_5$-Kohlenwasserstoffgemisch, welches löslichere Kohlenwasserstoffe als das konjugierte Diolefin und weniger lösliche Kohlenwasserstoffe als das konjugierte Diolefin enthält, einer extraktiven Destillation mit dem erfindungsgemäß zu verwendenden Lösungsmittelgemisch unterworfen wird, wobei ein die weniger löslichen Kohlenwasserstoffe enthaltendes Destillat und ein das konjugierte Diolefin und die löslicheren Kohlenwasserstoffe und das selektive Lösungsmittel enthaltender Extrakt abgetrennt werden. Aus dem Extrakt kann das konjugierte Diolefin als Rohprodukt isoliert werden, das für manche Verwendungszwecke eine ausreichende Reinheit aufweist, das jedoch auch noch weiteren Reinigungsoperationen, z.B. einer fraktionierten Destillation, unterworfen werden kann. Vorteilhaft wird das konjugierte Diolefin jedoch unter Anwendung von zwei hintereinandergeschalteten extraktiven Destillationsstufen unter Verwendung der erfindungsgemäß zu verwendenden Lösungsmittelmischung gewonnen.

Hierbei werden beispielsweise — wie bereits vorstehend beschrieben — in der ersten Stufe der extraktiven Destillation ein die weniger löslichen Kohlenwasserstoffe enthaltendes Destillat und ein das konjugierte Diolefin und die löslicheren Kohlenwasserstoffe und das selektive Lösungsmittel enthaltender Extrakt abgetrennt. Dieser Extrakt wird vom selektiven Lösungsmittel befreit, wobei ein Gemisch aus dem konjugierten Diolefin und den löslicheren Kohlenwasserstoffen erhalten wird. Dieses Gemisch wird einer zweiten extraktiven Destillation mit dem selektiven Lösungsmittel unterworfen, wobei das konjugierte Diolefin als Destillat und ein Extrakt erhalten werden, der die löslicheren Kohlenwasserstoffe und das selektive Lösungsmittel enthält. Der erhaltene Extrakt wird anschließend vom selektiven Lösungsmittel befreit, wobei ein die löslicheren Kohlenwasserstoffe enthaltender Kohlenwasserstoffstrom erhalten wird.

Als konjugierte Diolefine enthaltendes Kohlenwasserstoffgemisch, das als Ausgangsgemisch für das Verfahren der vorliegenden Erfindung geeignet ist, kann eine $C_4$- oder $C_5$-Fraktion, die durch thermisches Kracken einer Petroleumfraktion (z.B. LPG, Naphtha usw.) erhalten wurde, eine durch Dehydrierung von n-Butan und/oder n-Buten erhaltene Butadien enthaltende Fraktion und eine durch Dehydrierung von Isopentan und/oder Isoamylen erhaltene Isopren enthaltende Fraktion verwendet werden. Im allgemeinen enthält das $C_4$-Kohlenwasserstoffgemisch 1,3-Butadien als konjugiertes Diolefin, Butane, Buten-1, Buten-2-trans, Buten-2-cis, Isobuten, Vinylacetylen, Äthylacetylen, 1,2-Butadien und gegebenenfalls geringe Mengen $C_3$- und/oder $C_5$-Kohlenwasserstoffe. Das $C_5$-Kohlenwasserstoffgemisch enthält in der Regel Isopren, trans- und cis-1,3-Pentadien und Cyclopentadien als konjugierte Diolefine sowie Pentane, n-Pentene, Isoamylen, Cyclopenten, höhere Acetylene.

Beispielsweise ergibt die extraktive Destillation einer $C_4$-Fraktion zunächst ein die Butane und Butene enthaltendes Destillat sowie einen 1,3-Butadien, Äthylacetylen, Vinylacetylen und 1,2-Butadien enthaltenden Extrakt, der, wenn er einer weiteren extraktiven Destillation unterworfen wird, als Destillat 1,3-Butadien liefert, während der Extrakt Äthylacetylen, Vinylacetylen und 1,2-Butadien enthält. Aus dem Äthylacetylen, Vinylacetylen und 1,2-Butadien enthaltenden Extrakt werden diese Kohlenwasserstoffe in einem Ausgaser abgetrennt und das entgaste Lösungsmittel in die extraktive Destillation zurückgeführt. Das als Destillat erhaltene 1,3-Butadien kann anschließend zur Abtrennung von gegebenenfalls noch vorhandenen sehr geringen Mengen von $C_3$- und/oder $C_5$-Kohlenwasserstoffen einer fraktionierten Destillation unterworfen werden.

Die nachstehenden Beispiele dienen der weiteren Erläuterung der Erfindung :

Beispiel 1

Beispiel 1 veranschaulicht die einstufige extraktive Destillation eines $C_4$-Kohlenwasserstoffgemisches.

Einer bei 1 bar und 15 °C betriebenen Füllkörperkolonne mit 25 mm Innendurchmesser und 2,50 m Höhe werden am Sumpf 0,768 kg/h $C_4$-Kohlenwasserstoffgemisch der folgenden Zusammensetzung zugeführt :

| Zusammensetzung | Gew. % |
|---|---|
| i-Butan | 1,33 |
| n-Butan | 4,44 |
| Buten-1 | 11,65 |
| i-Buten | 28,21 |
| Buten-2-trans | 7,28 |
| Buten-2-cis | 4,45 |
| Butadien-1,3 | 41,98 |
| Butadien-1,2 | 0,31 |
| Äthylacetylen | 0,24 |
| Vinylacetylen | 0,11 |

Am Kopf der Kolonne werden 4,60 kg/h im Kreise geführtes selektives Lösungsmittel von 15 °C aus 90 Gew.% Dimethylformamid und 10 Gew.% Essigsäureäthylester zugegeben. 0,379 kg/h Raffinat mit einem Gehalt von 2 Gew.% Butadien-1,3 und 5,6 Gew.% Buten-2-cis werden am Kopf der Kolonne gasförmig abgezogen. Am Sumpf der Kolonne wird ein die leichter löslichen Kohlenwasserstoffe enthaltender Extrakt erhalten, der zur Ausgasung dem Kopf einer nachgeschalteten Kolonne mit 10 Glockenböden zugeführt wird, deren Sumpftemperatur auf 130 bis 140 °C gehalten wird. Vom Sumpf der Glockenbodenkolonne wird das weitgehend von den Kohlenwasserstoffen befreite selektive Lösungsmittel nach Abkühlung auf den Kopf der Füllkörperkolonne zurückgeführt. In der Mitte der Glockenbodenkolonne werden 0,389 kg/h Rohbutadien mit einem Butadien-1,3-Gehalt von 80,61 Gew.% und einem Buten-2-cis-Gehalt von 3,3 Gew.% abgezogen. Die am Kopf der Glockenbodenkolonne austretenden Kohlenwasserstoffe werden gasförmig in den Sumpf der Füllkörperkolonne zurückgeführt. Das Verhältnis L/G von Lösungsmittelumlauf L (4,60 kg/h) zu Einsatz an $C_4$-Kohlenwasserstoffgemisch G (0,768 kg/h) beträgt 5,99.

Vergleichsversuch

In einem Vergleichsversuch verfährt man wie im vorstehenden Beispiel 1 beschrieben, wobei jedoch als selektives Lösungsmittel reines Dimethylformamid verwendet wird und das $C_4$-Kohlenwasserstoffgemisch in einer gegenüber dem Beispiel 1 erniedrigten Menge von 0,621 kg/h am Sumpf der Füllkörperkolonne zugegeben wird, wobei 0,306 kg/h Raffinat und 0,315 kg/h Rohbutadien erhalten werden, d.h. die Aufteilung des $C_4$-Kohlenwasserstoffgemischs im Raffinat und Rohbutadien erfolgt praktisch im gleichen Verhältnis, wie im Beispiel 1. Die Schlüsselkomponenten Buten-2-cis und Butadien-1,3 werden im Beispiel 1 und im Vergleichsversuch im Raffinat und Rohbutadien auf die gleichen Gehalte abgereichert. Das Verhältnis L/G von Lösungsmittelumlauf L (4,60 kg/h) zu Einsatz G an $C_4$-Kohlenwasserstoffgemisch (0,621 kg/h) beträgt 7,41, d.h. im Vergleichsversuch war im Vergleich zum Beispiel ein um mehr als 23 % höheres L/G-Verhältnis erforderlich, um den gleichen Trennerfolg wie bei der Arbeitsweise gemäß Beispiel 1 zu erzielen.

Beispiel 2

Man verfährt wie in Beispiel 1 und dem zugehörigen Vergleichsversuch beschrieben, wobei jedoch in Beispiel 2 die Temperatur 5 °C höher liegt und als selektives Lösungsmittel eine Mischung aus 90 Gew.% N-Methylpyrrolidon und 10 Gew.% Kohlensäurediäthylester verwendet und ein Verhältnis L/G von Lösungsmittelumlauf L zu Einsatz an $C_4$-Kohlenwasserstoffgemisch G von 6,60 aufrecht erhalten wird und im Vergleichsbeispiel zu Beispiel 2 als selektives Lösungsmittel eine Mischung aus 91,7 Gew.% N-Methylpyrrolidon und 8,3 Gew.% Wasser angewendet und ein Verhältnis L/G von Lösungsmittelumlauf L zu Einsatz G an $C_4$-Kohlenwasserstoffgemisch von 12,74 aufrecht erhalten wird, d.h. um den gleichen Trennerfolg beim Vergleichsversuch zu Beispiel 2 wie bei der Arbeitsweise gemäß Beispiel 2 zu erzielen, war im Vergleichsversuch gegenüber dem Beispiel ein um 93 % höheres L/G-Verhältnis erforderlich.

Beispiel 3

Man verfährt wie in Beispiel 1 und dem zugehörigen Vergleichsversuch beschriebenen, wobei in Beispiel 3 als selektives Lösungsmittel eine Mischung aus 90 Gew.% N-Methylpyrrolidon und 10 Gew.% Essigsäureäthylester verwendet und ein Verhältnis L/G von Lösungsmittelumlauf L zu Einsatz an $C_4$-Kohlenwasserstoffgemisch G von 6,72 aufrecht erhalten wird und im Vergleichsbeispiel zu Beispiel 3 als selektives Lösungsmittel eine Mischung aus 91,7 Gew.% N-Methylpyrrolidon und 8,3 Gew.% Wasser angewendet und ein Verhältnis L/G von Lösungsmittelumlauf zu Einsatz G an $C_4$-Kohlenwasserstoffgemisch von 12,74 aufrecht erhalten wird. Während beim Beispiel 3, ausgehend von 0,658 kg/h an Einsatz G mit 4,18 Gew.% Buten-2-cis und 42,58 Gew.% Butadien-1,3 0,340 kg/h Raffinat mit 5,17 Gew.% Buten-2-cis und 2,47 Gew.% Butadien-1,3 sowie 0,318 kg/h Rohbutadien mit 3,24 Gew.% Buten-2-cis und 76,75 Gew.% Butadien-1,3 erhalten wurde, ergab sich im Vergleichsversuch trotz einer Erhöhung des L/G-Verhältnisses um fast 90 %, ausgehend von 0,471 kg/h Einsatz G mit 4,45 Gew.%

Buten-2-cis und 41,98 Gew.% Butadien-1,3 0,235 kg/h Raffinat mit nur 4,41 Gew.% Buten-2-cis und noch 14,77 Gew.% Butadien-1,3 sowie 0,236 kg/h Rohbutadien mit noch 4,66 Gew.% Buten-2-cis und nur 69,07 Gew.% Butadien-1,3.

**Ansprüche**

1. Verfahren zur Gewinnung eines konjugierten Diolefins aus einem dieses enthaltenden $C_4$- oder $C_5$-Kohlenwasserstoffgemisch durch ein- oder mehrstufige extraktive Destillation mit einem selektiven Lösungsmittel, dadurch gekennzeichnet, daß man als selektives Lösungsmittel eine Lösungsmittelmischung verwendet, die

a) 1 bis 99 Gewichtsprozent eines N-alkylsubstituierten niederen aliphatischen Säureamids oder eines N-alkylsubstituierten alicyclischen Säureamids mit 5 Ringgliedern, und

b) 1 bis 99 Gewichtsprozent eines niederen aliphatischen Carbonsäureesters oder Kohlensäureesters mit einem Siedepunkt im Bereich von 30 °C bis 200 °C enthält.

2. Verfahren nach Anspruch 1, dadurch gekennzeichnet, daß man eine Lösungsmittelmischung verwendet, die

a) 1 bis 99 Gewichtsprozent Dimethylformamid, Diäthylformamid, Dimethylacetamid, Diäthylacetamid, Formylmorpholin oder N-Methylpyrrolidon und

b) 1 bis 99 Gewichtsprozent eines niederen aliphatischen Carbonsäureesters oder Kohlensäureesters mit einem Siedepunkt im Bereich von 30 °C bis 200 °C enthält.

3. Verfahren nach Anspruch 1 und 2, dadurch gekennzeichnet, daß die Lösungsmittelmischung höchstens 5 Gewichtsprozent Wasser, bezogen auf die Lösungsmittelmischung enthält.

**Claims**

1. A process for isolating a conjugated diolefin from a $C_4$- or $C_5$-hydrocarbon mixture containing the diolefin, by single-stage or multi-stage extractive distillation using a selective solvent, characterized in that there is used as selective solvent a solvent mixture which contains

a) from 1 to 99 percent by weight of a N-alkyl-substituted lower aliphatic acid amide or of a N-alkyl-substituted alicyclic acid amide having 5 ring members, and

b) from 1 to 99 percent by weight of a lower aliphatic carboxylic acid ester or carbonic acid ester boiling at from 30 °C to 200 °C.

2. A process as claimed in claim 1, characterized in that a solvent mixture is used which contains

a) from 1 to 99 percent by weight of dimethylformamide, diethylformamide, dimethylacetamide, diethylacetamide, formylmorpholine or N-methylpyrrolidone, and

b) from 1 to 99 percent by weight of a lower aliphatic carboxylic acid ester or carbonic acid ester boiling at from 30 °C to 200 °C.

3. A process as claimed in claims 1 and 2, characterized in that the solvent mixture contains at most 5 percent by weight of water, based on the solvent mixture.

**Revendications**

1. Procédé d'extraction d'une dioléfine conjuguée d'un mélange d'hydrocarbures en $C_4$ ou $C_5$, qui la contient, à l'aide d'une distillation en un ou plusieurs stades dans un solvant sélectif, caractérisé en ce que le solvant sélectif est un mélange de solvants, formé :

a) de 1 à 99 % en poids d'un amide d'acide aliphatique inférieur, substitué sur l'atome d'azote par des radicaux alcoyle, ou d'un amide d'acide alicyclique pentagonal, substitué sur l'atome d'azote par des radicaux alcoyle, et

b) de 1 à 99 % en poids d'un ester d'un acide carboxylique aliphatique inférieur ou de l'acide carbonique avec un point d'ébullition dans la gamme de 30 à 200 °C.

2. Procédé suivant la revendication 1, caractérisé en ce que le mélange de solvants est composé :

a) de 1 à 99 % en poids de diméthyl-formamide, de diéthyl-formamide, de diméthyl-acétamide, de diéthyl-acétamide, de formyl-morpholine ou de N-méthyl-pyrrolidone et

b) de 1 à 99 % en poids d'un ester d'un acide carboxylique aliphatique inférieur ou de l'acide carbonique avec un point d'ébullition dans la gamme de 30 à 200 °C.

3. Procédé suivant l'une des revendications 1 et 2, caractérisé en ce que le mélange de solvants contient au maximum 5 % en poids d'eau par rapport au poids du mélange de solvants.